# EUROPEAN PATENT SPECIFICATION

(11) **EP 1 342 730 B1**
(45) Date of publication and mention of the grant of the patent: **15.03.2006**
(21) Application number: 01270554.7
(22) Date of filing: 10.12.2001
(51) Int. Cl.: C07K 19/00, C12N 15/00, C07K 14/505

(54) **FUSION PROTEIN HAVING THE ENHANCED IN VIVO ACTIVITY OF ERYTHROPOIETIN**
FUSIONSPROTEIN MIT VERBESSERTER IN VIVO ERYTHROPOIETINWIRKUNG
PROTEINE DE FUSION PRESENTANT UNE ACTIVITE I IN VIVO /I AMELIOREE D'ERYTHROPOIETINE

(30) Priority: 11.12.2000 KR 2000075230; 21.11.2001 KR 2001072713
(43) Date of publication of application: 10.09.2003
(73) Proprietor: Cheil Jedang Corporation, Seoul 100-095 (KR)
(72) Inventor: LEE, Dong-Eok, 138-746 Seoul (KR); OH, Myung-Suk, 467-812 Kyunggi-Do (KR); KIM, Ki-Wan, 120-848 Seoul (KR); CHUNG, Bo-Sup, Anyang-Shi, 430-013 Kyunggi-Do (KR); HA, Byung-Jhip, 449-718 Kyunggi-Do (KR); PARK, Ji-Sook, 139-220 Seoul (KR)
(74) Representative: Wichmann, Hendrik
(86) International application number: PCT/KR2001/002137
(87) International publication number: WO 2002/048194

(56) References cited:
- EP-A1- 0 640 619
- WO-A-01/02017
- WO-A-93/06844
- WO-A-94/24148
- US-A- 5 585 345
- US-A- 5 712 122
- US-A- 5 759 828
- US-A- 5 792 460

### Sequence Listing

<110> Cheil Jedang Corporation
<120> Fusion protein having the enhanced in vivo activity of erythropoietin
<130> PC01-0331-JIJD
<150> KR10-2000-0075230
   <151> 2000-12-11
<160> 10
<170> KopatentIn 1.71
<210> 1
   <211> 34
   <212> PRT
   <213> Homo sapiens
<220>
   <221 > PEPTIDE
   <222> (1)..(34)
   <223> Amino acids in the positions of 112 to 145 of human chorionic gonadotropin(HCG) beta subunit
<400> 1
   Asp Pro Arg Phe Gln Asp Ser Ser Ser Ser Lys Ala Pro Pro Pro Ser
   1 5 10 15
   Leu Pro Ser Pro Ser Arg Leu Pro Gly Pro Ser Asp Thr Pro Ile Leu
   20 25 30
   Pro Gln
<210> 2
   <211> 220
   <212> PRT
   <213> Artificial Sequence
<220>
   <223> Fusion protein(ECTP) of erythropoietin(EPO) and carboxy terminal peptide(CTP) of human chorionic gonadotropin(HCG) beta subunit
<400> 2
   Met Gly Val His Glu Cys Pro Ala Trp Leu Trp Leu Leu Leu Ser Leu
   1 5 10 15
   Leu Ser Leu Pro Leu Gly Leu Pro Val Leu Gly Ala Pro Pro Arg Leu
   20 25 30
   Ile Cys Asp Ser Arg Val Leu Glu Arg Tyr Leu Leu Glu Ala Lys Glu
   35 40 45
   Ala Glu Asn Ile Thr Thr Gly Cys Ala Glu His Cys Ser Leu Asn Glu
   50 55 60
   Asn Ile Thr Val Pro Asp Thr Lys Val Asn Phe Tyr Ala Trp Lys Arg
   65 70 75 80
   Met Glu Val Gly Gln Gln Ala Val Glu Val Trp Gln Gly Leu Ala Leu
   85 90 95
   Leu Ser Glu Ala Val Leu Arg Gly Gln Ala Leu Leu Val Asn Ser Ser
   100 105 110
   Gln Pro Trp Glu Pro Leu Gln Leu His Val Asp Lys Ala Val Ser Gly
   115 120 125
   Leu Arg Ser Leu Thr Thr Leu Leu Arg Ala Leu Gly Ala Gln Lys Glu
   130 135 140
   Ala Ile Ser Pro Pro Asp Ala Ala Ser Ala Ala Pro Leu Arg Thr Ile
   145 150 155 160
   Thr Ala Asp Thr Phe Arg Lys Leu Phe Arg Val Tyr Ser Asn Phe Leu
   165 170 175
   Arg Gly Lys Leu Lys Leu Tyr Thr Gly Glu Ala Cys Arg Thr Gly Asp
   180 185 190
   Ser Ser Ser Ser Lys Ala Pro Pro Pro Ser Leu Pro Ser Pro Ser Arg
   195 200 205
   Leu Pro Gly Pro Ser Asp Thr Pro Ile Leu Pro Gln
   210 215 220
<210> 3
   <211> 29
   <212> DNA
   <213> Artificial Sequence
<220>
   <223> Primer EP1 having the nucleotide sequence complementary to the terminal sequence of EPO cDNA
<400> 3
   atgggggcac gaatgtcctg cctggctgg 29
<210> 4
   <211> 20
   <212> DNA
   <213> Artificial Sequence
<220>
   <223> Primer EC2 having the nucleotide sequence complementary to the terminal sequence of EPO cDNA
<400> 4
   gtcccctgtc ctgcaggcct 20
<210> 5
   <211> 35
   <212> DNA
   <213> Artificial Sequence
<220>
   <223> HCG beta subunit CTP gene fragment EC1
<400> 5
   aggggaggcc tgcaggacag gggactcctc ttcct   35
<210> 6
   <211> 35
   <212> DNA
   <213> Artificial Sequence
<220>
   <223> HCG beta subunit CTP gene fragment C2
<400> 6
   ggaaggctgg ggggaggggc ctttgaggaa gagga 35
<210> 7
   <211> 35
   <212> DNA
   <213> Artificial Sequence
<220>
   <223> HCG beta subunit CTP gene fragment C3
<400> 7
   ccagccttcc aagcccatcc cgactcccgg ggccc 35
<210> 8
   <211> 35
   <212> DNA
   <213> Artificial Sequence
<220>
   <223> HCG beta subunit CTP gene fragment C4
<400> 8
   ttattgtggg aggatcgggg tgtccgaggg ccccg 35
<210> 9
   <211> 25
   <212> DNA
   <213> Artificial Sequence
<220>
   <223> Primer EP11 for PCR to obtain the desired fusion gene ECTP
<400> 9
   taagcttatg ggggtgcacg aatgt 25
<210> 10
   <211> 28
   <212> DNA
   <213> Artificial Sequence
<220>
   <223> Primer EP22 for PCR to obtain the desired fusion gene ECTP
<400> 10
   tggatcctta ttgtgggagg atcggggt 28
<210> 11
   <211> 226
   <212> PRT
   <213> Artificial Sequence
<220>
   <223> Fusion protein (ECTP) of erythropoietin (EPO) and carboxy terminal peptide AA 1-34 (CTP) of human chorionic gonadotropin(HCG) beta subunit
<400> 2

## Description

The present invention relates to a fusion protein having the enhanced *in vivo* activity of erythropoietin that is a novel medicine for the treatment of anemia. More specifically, the present invention relates to a fusion protein that has the highly enhanced *in vivo* activity of erythropoietin by fusion of EPO molecule with a particular peptide that has half-life elongating activity and is derived from the human body.
Erythropoietin (EPO), a glycoprotein having the molecular weight of 30,000 to 34,000, stimulates production of a red blood cell. This protein starts its functions by binding with receptors on erythrocyte precursor cells to result in increase of calcium ion concentration in a cell, increase of biosynthesis of DNA, and stimulation for the formation of hemoglobin and the like. Recombinant human EPO (rhEPO) has been used for the treatment of anemia from renal failure, anemia of a premature baby, anemia from hypothyroidism, anemia from malnutrition, and the like. On the other hand, a clinical use of rhEPO is on the increase. However, the use of rhEPO may cause inconvenience and high costs from the administration of on average 3 times a week due to the short half-life of the rhEPO. Thus, if the *in vivo* activity of EPO is maintained for a long time, the administration frequency of EPO may be decreased very much.

*In vivo* activity of EPO is proportional to the *in vivo* half-life of EPO. It is known that *in vivo* half-life of EPO is correlated to the content of sialic acid that is located at the terminal of carbohydrate chains of EPO. Therefore, *in vivo* activity of EPO is highly dependent on the content of carbohydrate chains. On the other hand, the forms of carbohydrate are different according to the kinds of the cells in which EPO is expressed, and therefore, the same glycoproteins may show different carbohydrates if they are expressed in different cells. Some bacteria, for example *E. coli,* are known not to be capable of attaching the carbohydrate chains to the protein. Generally, proteins expressed in *E. coli* do not contain the carbohydrate chains, and thus, *E. coli*-derived EPO, which does not contain the carbohydrate chains, exhibits both enhanced *in vitro* activity and decreased *in vivo* activity. Deglycosylated EPO is rapidly eliminated from the human body and has an extremely short half-life. In conclusion, the carbohydrate chains play a very important role in the activity of EPO.

Many studies have been conducted to enhance the activity of EPO. The main method is substitution of some amino acids of EPO by mutagenesis. For example, PCT/US94/09257 filed by Amgen, and titled "Erythropoietin Analog", discloses a method to increase the half-life of EPO by increasing the carbohydrate contents using mutagenesis. Also, an attempt was made to increase the half-life of EPO by formation of EPO dimer. See, A. J. Sytkowski et al., *J.B.C.* vol. 274, No. 35, pp24773-24778. Besides, another known method is to enhance *in vivo* activity of EPO by fusing new amino acids, peptides or protein fragment with EPO and increasing the carbohydrate content, i.e., sialic acid content of EPO. However, all amino acids, peptides or heterogeneous protein fragments may not be used in such methods. In most cases, such fusions result in decrease or loss of inherent activity of protein and may cause a problem of antigenicity when used *in vivo.*

EP-A1 0 640 619 discloses EPO fusion proteins that differ from the fusion protein shown in Figure 2 by the presence of an arginine residue between the EPO and CTP amino acid sequence.

Fusion proteins or chimeric proteins, etc. have been studied, for example, for follicle stimulating hormone, a sex hormone. See, Furuhashi et al., 1995, *Mol. Endocrinol*.). However, the methods have not been applied to industry since protein modification itself has many risks, and the target protein is not readily obtained without professional skills, and the inherent activity of protein may be decreased or lost to cause the opposite result.

The present inventors have extensively studied new methods for enhancing the *in vivo* activity of EPO by fusing new amino acids, peptides or protein fragment with EPO. As a result, the present inventors have revealed that a fusion protein of EPO with a peptide fragment located at the carboxy terminal (hereinafter, called as "CTP") of β subunit of human chorionic gonadotropin (hereinafter, called as "HCG") has a highly enhanced half-life due to a lot of amino acids that increase glycosylation site without loss of the inherent activity of EPO, and did not cause any antigenicity when applied to the human body. Therefore, the inventors have completed the present invention.

The object of the present invention is to provide a fusion protein having enhanced *in vivo* activity of human EPO, containing CTP of HCG β subunit fused with human EPO at the carboxy terminal of human EPO.

Another object of the present invention is to provide nucleotide sequences encoding the fusion protein, a plasmid containing the nucleotide sequences, and a host cell line transfected with the plasmid.

Moreover, the present invention is providing a process for the preparation of the fusion protein having the enhanced *in vivo* activity of human EPO by comprising cultivating the above transfected cell line. First, the present invention relates to a fusion protein having an enhanced *in vivo* activity of human EPO, containing CTP of HCG β subunit fused with human EPO at the carboxy terminal of human EPO. The CTP preferably comprises the amino acid sequence of SEQ ID No. 1 that discloses amino acids of positions 112 to 145, particularly 118 to 145 of HCG β subunit.

Preferably, the fusion protein according to the present invention comprises the amino acid sequence of SEQ ID No. 2.

Second, the present invention relates to nucleotide sequences encoding the fusion protein, a plasmid containing the nucleotide sequences, and a host cell line, preferably CHO (Chinese hamster ovary) cell, transfected with the plasmid.

Third, the present invention relates to a process for the preparation of a fusion protein having an enhanced *in vivo* activity of human EPO by cultivating the above transfected cell line.

Below, the present invention will be explained in detail.

The present invention comprises the steps of preparation and cloning of human EPO cDNA, construction of an expression vector incorporating EPO-encoding DNA, transfection of animal cells, expression of human EPO, purification of the expressed human EPO, and biological assays thereof

### (1) Preparation and cloning of human EPO cDNA

EPO cDNA may be obtained by performing the well-known RT-PCR technique (PreMix Kit™ of Bioneer) using previously prepared EP1 and EC2 primers that are complementary to the both terminals of EPO cDNA in cDNA library of human-derived fetal liver (Invitrogen):

Obtained EPO cDNA is cloned to pGEM-T (Promega), a cloning vector, which is designated as pGEMT-EPO. The pGEMT-EPO is then sequenced, and used as a probe for the following operations.

CTP gene of HCG β unit used in the present invention may be obtained by synthesis and self-priming PCR. Synthesized oligo-nucleotide fragments are EC1, C2, C3 and C4:

1µl (50 pmole/µl) of each 4 oligomers is taken and PCR is performed using a high fidelity Tag system (BM) for these oligomers.

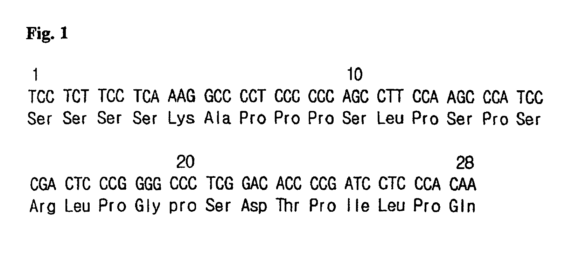

About 100 bps of the gene fragment (CTP) are identified in 1% agarose gel. This gene encodes the 28 amino acids (amino acids of positions 118 to 145) of the carboxy terminal of HCG β subunit (Refer to Fig. 1).

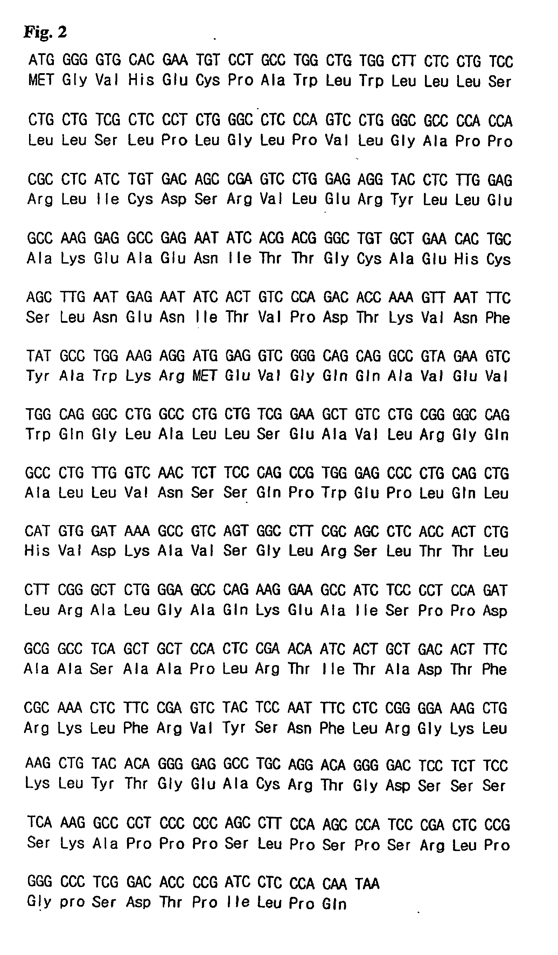

A PCR is performed using pGEMT-EPO as a probe and EP1 and EC2 as primers to obtain EPO gene only. The PCR is also performed using both the obtained EPO gene and CTP gene as templates, and EP1 and C4 as primers by a high fidelity Tag system (BM), to obtain 630 bps of the intended fusion gene, ECTP gene. This gene is cloned to a cloning vector, pGEM-T (reverse direction) and then sequenced (designated as pGEMT-ECTP, refer to Fig.2).

### (2) Construction of expression vector incorporating EPO-encoding DNA

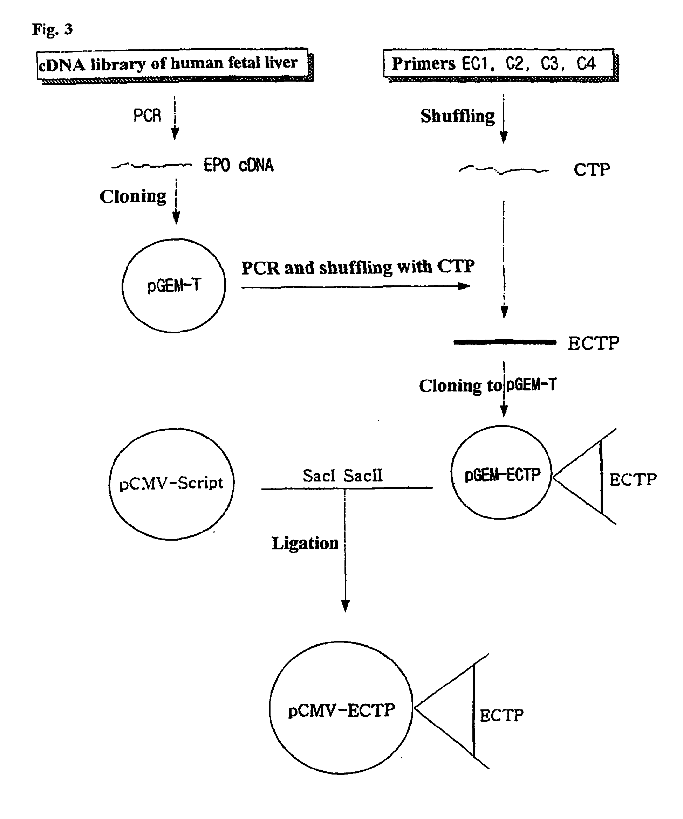

pCMV-Script(Stratagene) vector or pcDNA3.1 vector(Invitrogen) are used as an expression vector.
(i) When pCMV-Script is used as an expression vector
   pCMV-Script and pGEMT-ECTP are treated with restriction enzymes *Sac* I and *Sac* II, respectively, and the linearized pCMV-Script and ECTP genes are purified using Qiagen gel extraction Kit on agarose gel. After ligation, the ligate was introduced into *E. coli* NM522. Plasmids are isolated from the colonies that are cultivated for one night in LB-kanamycine solid medium, and are treated with restriction enzymes *Sac* I and *Sac* II. Colonies having ECTP gene are then screened by 1% agarose gel electrophoresis. This plasmid is designated as pCMV-ECTP(refer to Fig.3).
(ii) When pcDNA3.1 is used as an expression vector
   The PCR is performed using pGEMP-ECTP as a template, and EP11 and EP22 as primers by the high fidelity Tag system (BM), to obtain 630 bps of the intended fusion gene, ECTP gene:

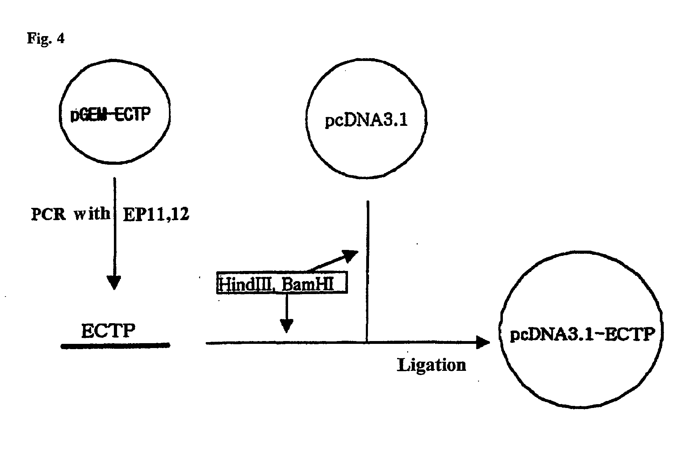

The terminals of ECTP gene have restriction sites of *Hind*III and *Bam*HI*,* respectively. pcDNA3.1 and the obtained ECTP gene are treated with restriction enzymes *Hind*III and *Bam*HI*,* respectively. The linearized pCMV-Script and ECTP genes are obtained using Qiagen gel extraction Kit on agarose gel, and ligated and introduced into *E*. *coli* NM522 to obtain transformed *E. coli.* Plasmids are isolated from the colonies that are cultivated for one night in LB-ampicillin solid medium, and are treated with restriction enzymes *Hind*III and *Bam*HI*.* Colonies having ECTP gene are then screened by 1% agarose gel electrophoresis. This plasmid is designated as pcDNA3.1-ECTP (Refer to Fig.4).

### (3) Transformation of CHO cell and the expression of ECTP

(i) When pCMV-Script is used as an expression vector
   CHO (tk-) cells are prepared by cultivating the CHO cells to reach 40-80 % confluency (1~4 x 10⁵ cells/60 mm dish) at 60 mm cell culture dish. 3µl of superfection reagent (BM) and 97µl of cell medium (α-MEM with media, no serum, no antibiotics) are mixed well. The plasmid pCMV-ECTP DNA (> 0.1µg/µl, about 2µg) is added to this mixture, and reacted for 5~10 minutes at room temperature, and then, added to the above-prepared cells. The medium is refreshed with G418 medium (α-MEM media, 10% serum, and 500µg/ml of G148) after 1 day. The medium is then refreshed with the G418 medium every day for 7∼10 days, and then cells without G418 resistance gene and negative control cells are dead. The selected cells on the G418 medium are sufficiently cultivated and the expressed ECTP protein is identified using EPO ELISA Kit (BM).
(ii) When pcDNA3.1 is used as an expression vector
   CHO (DG44) cells are prepared by cultivating the CHO cells to reach 40-80 % confluency (1∼4 x 10⁵ cells/ 60 mm dish) at 60 mm cell culture dish. 3µl of superfection reagent (BM) and 97µl of cell medium (α-MEM media, no serum, no antibiotics) are mixed well, and plasmid pcDNA3.1-ECTP DNA (>0.1µg/µl, about 2µg) and pLTRdhfr26(ATCC37295, 0.2 µg) are added to the obtained mixture, reacted for 5∼10 minutes at room temperature, and then, added to the above-prepared cells. The medium is refreshed with the G418 medium (α-MEM with media, 10% FBS) after 1 day. The medium is then refreshed with the G418 medium every day for 7∼10 days to kill cells without G418 resistance gene and negative control cells. The selected cells on G418 medium are cultivated sufficiently and the expressed ECTP protein is identified using EPO ELISA Kit (BM).

### (4) Purification of the expressed ECTP

Affinity resin for the isolation and purification is prepared using anti-EPO monoclonal antibody (R&D Systems) as follows:

0.3g of CNBr-activated Sepharose CL 4B is swelled for 20 minutes in 1mM HCl, and the resin is moved to a column, and washed with 1mM HCl. The resin is washed with 4 ml of coupling buffer (0.1 M NaHCO₃, 0.5M NaCl, pH 8.3), and immediately mixed with anti-EPO monoclonal antibody (500ug/ vial) contained in 4 ml of coupling buffer in a tube, and reacted at room temperature for 2 hours with stirring the tube. Refreshed with Blocking Buffer (0.2 M glycine, pH 8.0), the resin reacts at room temperature for 2 hours with stirring the tube. Then, the tube is washed with 6.5 ml of coupling buffer, 6.5 ml of acetate buffer (0.1 M acetic acid, 0.5 M NaCl, pH 4) and 6.5 ml of coupling buffer in turn. After the column is prepared, purification of the expressed ECTP is performed as described below.

The cells are cultivated in serum free medium for 1 day, and the medium without cells is concentrated to about 5 folds using Centriprep™ ( Millipore, MWCO 10,000). This is then loaded to the column equilibrated with PBS at the flow rate of 20 ml/hr, and washed again with PBS. The column is then washed with the dissolution buffer (0.1M glycine, pH 2.8) and the eluent is immediately titrated with 1M Tris-HCl to pH 7.5. The purity is at least 97% when analyzed by SDS-PAGE and silver staining.

### (5) Measurement of activity by the Bioassay method and biochemical analysis

*In vitro* biological activities of the expressed and properly purified EPO and ECTP are measured using mouse spleen cells treated with phenylhydrazine. The activity of ECTP is shown to be higher than that of EPO, which demonstrates that the attached CTP in the ECTP may not inhibit the activity of EPO.

IEF (Isoelectric focusing) is performed to ensure the existence of carbohydrate, that is, sialic acid that is located at the terminal of carbohydrate and is known to play an important role in the *in vivo* activity of EPO. The increase of sialic acid content will move the protein to the low pH due to the strong negative charge of sialic acid. IEF gel patterns (Novex) demonstrate that the ECTP moves to the lower pH than the EPO. This indicates that the *in vivo* activity of ECTP may be far more prolonged than the activity of EPO.

### (6) Pharmacokinetic experiments

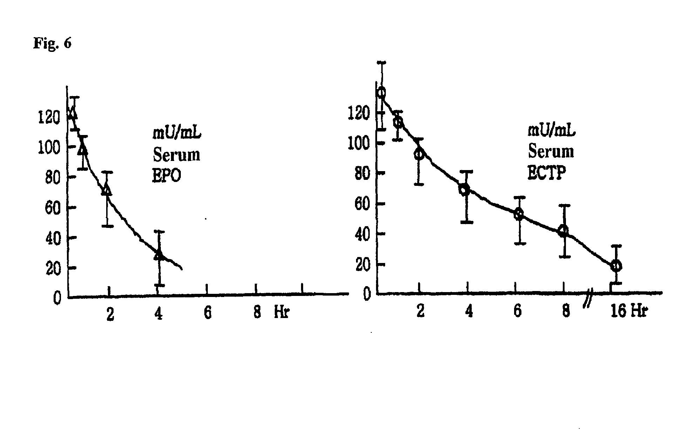

Pharmacokinetic experiments are performed to test whether the prepared candidate substance has prolonged *in vivo* activity. The candidate substance is intravenously administrated to 4 mice at the amount of 20 units/mouse. After sampling blood from the mice, the concentrations of EPO and ECTP are determined using an EIA Kit of Boehringer Manheim. The candidate substance, ECTP, shows a far longer half-life than the control, EPO (Refer to Fig.6).

### Brief Explanation of the Drawings

Fig. 1 shows the base and amino acid sequences of carboxy terminal peptide (CTP) of HCG;
Fig. 2 shows the base and amino acid sequences of the ECTP that is a fusion protein of EPO and CTP;
Fig. 3 is a scheme showing the procedures to prepare the expression vector pCMV-ECTP;
Fig. 4 is a scheme showing the procedures to prepare the expression vector pcDNA3.1-ECTP;
Fig 5a is an electrophoresis photograph of purified ECTP;
Fig 5b is an IEF photograph of expressed EPO and ECTP; and
Fig 6 is a graph showing the pharmacokinetic results of EPO and ECTP.

The present invention will be explained in more detail with reference to the following examples. However, the scope of the present invention is not or should not be limited to the examples.

### Example 1: Preparation and cloning of human EPO cDNA

EPO cDNA was obtained by performing the well-known RT-PCR technique (PreMix Kit™ of Bioneer) in the cDNA library of the human-derived fetal liver (Invitrogen) using previously prepared EP1 and EC2 primers that are complementary to both terminals of EPO cDNA. PCR was performed in 30 cycles under the condition of 55°C for 35 seconds for annealing, 72°C for 40 seconds, and 94°C for 20 seconds to obtain EPO cDNA. The obtained EPO cDNA was cloned to pGEM-T(Promega), a cloning vector. That is, the PCR products were run on 1% agarose gel, and the separated EPO cDNA was ligated into pGEM-T. The recombined pGEM-T was then introduced into *E. coli* NM522 to obtain transformed *E. coli.* Plasmid DNAs were purified from the white colonies that are cultivated for one night in LB-ampicilline solid medium containing X-gal/IPTG, and were treated with restriction enzymes *Sac* I and *Sac* II to screen the colonies containing EPO cDNA. The obtained plasmid is designated as pGEMT-EPO.

The CTP gene of HCG β unit was obtained by nucleotide synthesis and self-priming PCR Synthesized oligonucleotide were EC1, C2, C3 and C4. 1µl(50 pmole/µl) of each 4 gene was taken by and PCR was performed in 15 cycles under the conditions of 55°C for 40 seconds for annealing, 72° for 40 seconds, and 94°C for 20 seconds. About 100 bps of DNA fragment were identified on 1% agarose gel. This gene encodes the 28 amino acids of the carboxy terminal of HCG β subunit (Refer to Fig. 1).

The PCR was performed under the same condition as described above using pGEMT-EPO as a template and EP1 and EC2 as primers to obtain EPO gene only. ECTP gene of 630 bps was obtained by performing the PCR in 30 cycles under the conditions of 58°C for 40 seconds for annealing, 72°C for 60 seconds, and 94°C for 20 seconds using EPO together with CTP as templates, and EP1 and EC2 as primers. This gene was cloned to the cloning vector pGEM-T in the same manner as mentioned above, and then sequenced (designated as pGEMT-ECTP, refer to Fig.2).

### Example 2: Construction of an expression vector pCMV-ECTP

pCMV-Script(Stratagene) was used as an expression vector. pCMV-Script and pGEMT-ECTP were treated with restriction enzymes *Sac* I and *Sac* II, respectively, and the linearized pCMV-Script and ECTP genes were obtained by the Qiagen gel extraction Kit on agarose gel. After ligation, the ligate was introduced into *E. coli* NM522. Plasmids were isolated from the colonies that were cultivated for one night in LB-kanamycine solid medium, and were treated with restriction enzymes *Sac* I and *Sac* II. The colonies having ECTP gene were then screened by 1% agarose gel electrophoresis. This plasmid is designated as pCMV-ECTP (Refer to Fig.3).

### Example 3: Construction of an expression vector pcDNA3.1-ECTP

pcDNA3.1 (Invitrogen) was used as an expression vector. PCR was performed using the pGEMP-ECTP as a template, EP11 and EP22 as primers, and high fidelity Tag system (BM), to obtain 630 bps of the intended fusion gene. The terminals of the ECTP gene have restriction sites for *Hind*III and *Bam*HI*,* respectively. pcDNA3.1 and the obtained ECTP gene were treated with restriction enzymes *Hind*III and *Bam*HI*,* respectively. The linearized pCMV-Script and ECTP genes were obtained by the Qiagen gel extraction Kit on agarose gel. After ligation, the ligate was introduced into *E. coli* NM522. Plasmids were isolated from the colonies that are cultivated for one night in LB-ampicillin solid medium, and were treated with restriction enzymes *Hind*III and *Bam*HI*.* The colonies containing ECTP gene were then screened by 1% agarose gel electrophoresis. This plasmid is designated as pcDNA3.1-ECTP (Refer to Fig.4).

### Example 4: Transformation of CHO cell and the expression of ECTP

(i) When pCMV-Script is used as an expression vector
   CHO (tk-) cells were prepared by cultivating the CHO cells to reach 40-80 % confluency (1~4 x 10⁵ cells/ 60 mm dish) at 60 mm cell culture dish. 3µl of superfection reagent (BM) and 97µl of cell medium (α-MEM media, no serum, no antibiotics) were mixed well and plasmid pCMV-ECTP DNA (> 0.1µg/µl, about 2µg) was added to this mixture and reacted for 5~10 minutes at room temperature, and then, added to the above-prepared cells. After one day, the medium was refreshed with the G418 medium (α-MEM media, 10% serum; and 500 µg/ml of G418). The medium was then refreshed with the G418 medium every day for 7~10 days to kill cells without G418 resistance gene and negative control cells. The selected cells on G418 medium were sufficiently cultivated and the expressed ECTP protein was identified using EPO ELISA Kit (BM).
(ii) When pcDNA3.1 is used as an expression vector
   CHO (DG44) cells were prepared by cultivating the CHO cells to reach 40-80 % confluency (1∼4 x 10⁵ cells/ 60 mm dish) at a 60 mm cell culture dish. 3µl of superfection reagent (BM) and 97µl of cell medium (α-MEM with media, no serum, no antibiotics) were mixed well and plasmid pcDNA3.1-ECTP DNA (> 0.1µg/µl, about 2µg) and pLTRdhfr26(ATCC37295, 0.2 µg) were added to the obtained mixture and reacted for 5~10 minutes at room temperature, and then, added to the above-prepared cells. The medium was refreshed with G418 medium (α-MEM with media, 10% FBS) after 1 day. The medium was then refreshed with the G418 medium every day for 7~10 days to kill cells without G418 resistance gene and negative control cells. The selected cells on G418 medium were sufficiently cultivated and the expressed ECTP protein were identified using EPO ELISA Kit (BM).

### Example 5: Purification of expressed ECTP

Affinity resin for the isolation and purification was prepared by the following procedures using an anti-EPO monoclonal antibody (R&D Systems).

0.3g of CNBr-activated Sepharose CL 4B was swelled for 20 minutes in 1mM HCl, and the resin was moved to the column, and washed with 1mM HCl. The resin was washed with 4 ml of coupling buffer (0.1 M NaHCO₃, 0.5M NaCl, pH 8.3), and immediately mixed with the anti-EPO monoclonal antibody (500µg/ vial) contained in 4 ml of the coupling buffer in a tube, and reacted for 2 hours at room temperature with stirring the tube. Substituting the buffer with Blocking Buffer (0.2 M glycine, pH 8.0), the reaction was continued for 2 hours at room temperature with stirring the tube. Then, the tube was washed with 6.5 ml of the coupling buffer, 6.5 ml of the acetate buffer (0.1 M acetic acid, 0.5 M NaCl, pH 4) and 6.5 ml of the coupling buffer in turn. After the column was prepared, purification of the expressed ECTP was performed as described below.

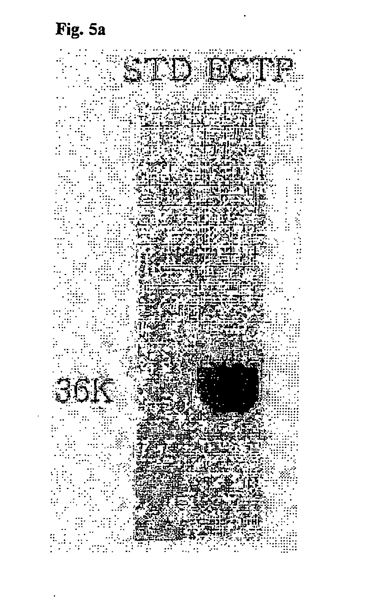

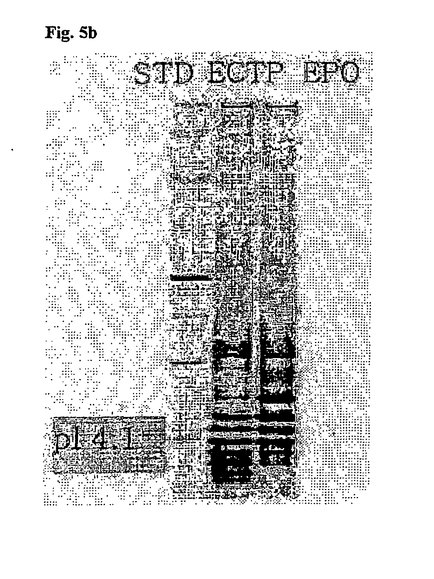

The cells were cultivated on serum free medium for 1 day, and the medium without cells were concentrated to about 5 folds using Centriprep™ (Millipore, MWCO 10,000). This was then loaded to the column equilibrated with PBS at 20 ml/hr of flow rate, and washed again with PBS. The column was then washed with a dissolution buffer (0.1M glycine, pH 2.8) and the eluent is immediately titrated with 1M Tris-HCl to pH 7.5. SDS-PAGE of the purifed ECTP was shown in Fig. 5a. The purity was at least 97% when analyzed by SDS-PAGE and silver staining.

### Example 6: Measurement of activity by the bioassay method and IEF analysis

Phenylhydrazine was injected to the mice twice a day for 2 days at the dose of 60 mg/kg. After 3 days, enlarged spleen was separated and ground with a homogenizer to obtain spleen cells. The spleen cells were diluted to 6×10⁶ cells/ml and 100 µl of the cells were added into 96 well plates. 0~500 mU/ml of standard EPO, and 104 mU/ml of the expressed EPO and ECTP were added to each well and the plate was placed at 37°C for 22 hours in the CO₂ incubator. 50 µl of dimethyl³[H]-thymidine(20µCi/ml) was added to the plate and further reacted for 2 hours in the same incubator. Each well was then adsorbed onto glass filters (Nunc 1-73164). The filters were washed with physiological saline 3 times and the amount of radioactivity of each filter was measured using β counter. When activities of the moderately purified EPO and ECTP are measured, the activity of ECTP was shown to be comparable to or higher than the activity of EPO, which demonstrated that the CTP attached to EPO might not inhibit the activity of EPO.

The IEF (soelectric focusing) analysis was performed to ensure the existence of the carbohydrate chains and sialic acids that is located at the terminal of carbohydrate and is known to play an important role in the *in vivo* activity of EPO. IEF gel patterns (Novex) were shown in Fig. 5b. As shown in Fig. 5b, it was shown that the ECTP moved to the negative pole more than EPO. This indicates that the *in vivo* activity of ECTP may be far more prolonged than the activity of EPO.

### Example 7: Pharmacokinetic experiments

Pharmacokinetic experiments for the mice were performed to test whether the prepared candidate substance has prolonged *in vivo* activity. The fusion protein purified by the method of Example 6 was administrated intravenously to 4 mice at the amount of 20 units/mouse. After sampling blood from the mice, concentrations of EPO and ECTP were determined at the interval of 30 minutes for the first 2 hours, and then at the interval of 2 hours using EIA Kit of Boehringer Manheim. The results were shown in Fig. 6. As shown in Fig. 6, the candidate substance, ECTP has a 2.5 times longer half-life than the control, EPO.

The fusion protein according to the present invention has an increased half-life of EPO without influencing the inherent activity of EPO due to presence of the amino acids that increase the carbohydrate chains, and do not cause the problem of antigenicity since CTP is an internal peptide, and thus, is industrially useful.

## Claims

1. A fusion protein of human EPO, with the carboxy terminal peptide (CTP) of human chorionic gonadotropin (HCG) β subunit, consisting of the amino acid sequence of SEQ ID NO.2.

2. A fusion protein of human EPO, with the carboxy terminal peptide (CTP) of human chorionic gonadotropin (HCG) β subunit, consisting of the amino acid sequence of SEQ ID NO. 11.

3. Nucleotide sequences encoding the fusion protein according to any one of the claims 1 to 2.

4. A process for preparation of a fusion protein having enhanced *in vivo* activity of human EPO comprising cultivation of a host cell line transfected with recombined vector containing the DNA according to the claim 3.

## Patentansprüche

1. Fusionsprotein des humanen EPO mit dem carboxy-terminalen Peptid (CTP) der β-Untereinheit von humanem chorionischen Gonadotropin (HCG), bestehend aus der Aminosäuresequenz von SEQ ID NR.2.

2. Fusionsprotein des humanen EPO mit dem carboxy-terminalen Peptid (CTP) der β-Untereinheit von humanem chorionischen Gonadotropin (HCG), bestehend aus der Aminosäuresequenz von SEQ ID NR.11.

3. Nukleotidsequenzen kodierend für das Fusionsprotein nach einem beliebigen der Ansprüche 1 bis 2.

4. Verfahren zur Herstellung eines Fusionsproteins, das erhöhte *in vivo*-Aktivität von humanem EPO besitzt, umfassend das Kultivieren einer Wirtszelllinie, die mit rekombiniertem Vektor transfiziert ist, der die DNA nach Anspruch 3 enthält.

## Revendications

1. Protéine de fusion de l'EPO humaine, avec le peptide carboxy-terminal (CTP) de la sous-unité β de la gonadotrophine chorionique humaine (HCG), consistant en la séquence d'acides aminés de SEQ ID N° : 2.

2. Protéine de fusion de l'EPO humaine, avec le peptide carboxy-terminal (CTP) de la sous-unité β de la gonadotrophine chorionique humaine (HCG), consistant en la séquence d'acides aminés de SEQ ID N° : 11.

3. Séquences nucléotidiques codant pour la protéine de fusion selon l'une quelconque des revendications 1 à 2.

4. Procédé de préparation d'une protéine de fusion possédant une activité d'EPO humaine *in vivo* améliorée comprenant la culture d'une lignée cellulaire hôte transfectée avec un vecteur recombiné contenant l'ADN selon la revendication 3.
